(19) Europäisches Patentamt
European Patent Office
Office européen des brevets

(11) EP 1 991 566 B1

(12) EUROPEAN PATENT SPECIFICATION

(45) Date of publication and mention of the grant of the patent: **11.03.2015 Bulletin 2015/11**

(21) Application number: **06843736.7**

(22) Date of filing: **27.12.2006**

(51) Int Cl.: ***C07K 14/395*** (2006.01)

(86) International application number: **PCT/JP2006/326365**

(87) International publication number: **WO 2007/099695 (07.09.2007 Gazette 2007/36)**

(54) **GENE ENCODING PROTEIN RESPONSIBLE FOR FLOCCULATION PROPERTY OF YEAST AND USE THEREOF**

GEN, DAS FÜR EIN FÜR DIE FLOCCULATIONSEIGENSCHAFT VON HEFE VERANTWORTLICHES PROTEIN CODIERT, UND VERWENDUNG DAVON

GÈNE CODANT POUR UNE PROTÉINE RESPONSABLE DES PROPRIÉTÉS DE FLOCULATION D'UNE LEVURE ET APPLICATIONS

(84) Designated Contracting States: **AT BE BG CH CY CZ DE DK EE ES FI FR GB GR HU IE IS IT LI LT LU LV MC NL PL PT RO SE SI SK TR**

(30) Priority: **28.02.2006 JP 2006053938**

(43) Date of publication of application: **19.11.2008 Bulletin 2008/47**

(73) Proprietor: **Suntory Holdings Limited**
**Kita-ku, Osaka-shi,**
**Osaka 530-8203 (JP)**

(72) Inventors:
- **NAKAO, Yoshihiro**
  **Mishima-gun, Osaka 6188503 (JP)**
- **KODAMA, Yukiko**
  **Mishima-gun, Osaka 6188503 (JP)**
- **SHIMONAGA, Tomoko**
  **Shimamoto-cho,**
  **Mishima-gun,**
  **Osaka 6188503 (JP)**

(74) Representative: **Vossius & Partner**
**Siebertstrasse 3**
**81675 München (DE)**

(56) References cited:
**WO-A-99/31269**

- **BROWN AND BUSSEY: "THE YEAST KRE9 GENE ENCODES AN O GLYCOPROTEIN INVOLVED IN CELL SURFACE BETA-GLUCAN ASSEMBLY" MOLECULAR AND CELLULAR BIOLOGY, WASHINGTON, DC, US, vol. 13, no. 10, October 1993 (1993-10), pages 6346-6356, XP002104903 ISSN: 0270-7306**
- **VERSTREPEN K J ET AL: "Yeast flocculation: What brewers should know" APPLIED MICROBIOLOGY AND BIOTECHNOLOGY, SPRINGER VERLAG, BERLIN, DE, vol. 61, no. 3, May 2003 (2003-05), pages 197-205, XP002426016 ISSN: 0175-7598**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### TECHNICAL FIELD

The present invention relates to a gene as defined in the claims encoding a protein responsible for flocculation property of yeast and use thereof, in particular, a brewery yeast for producing alcoholic beverages with appropriate flocculation property and a method for producing said beverages. More particularly, the present invention relates to a yeast, which shows appropriate flocculation property by controlling expression level of non-Sc KRE9 gene encoding a protein responsible for flocculation property of brewery yeast, especially non-ScKRE9 gene specific to a lager brewing yeast, to a method for selecting said yeast, to a method for breeding said yeast and to a method for producing alcoholic beverages with said yeast.

### BACKGROUND ART

Flocculation property of yeast used for fermentation in alcoholic beverages is very important Flocculation property of yeast means property of forming aggregation as a result of interaction among each individual yeast cells, thus sedimenting to the bottom of the liquid in a liquid culture medium.

For example, yeasts used for fermentation of lager-type beer, which is popularly drunk at the present day, are also called bottom fermenting yeast, because the yeast have a character of flocculating and sedimenting to the bottom of fermentation broth near the end of fermentation. In beer brewing, yeast is recovered after fermentation and the recovered yeast is used at the subsequent fermentation, which is called "Renjo" which means successive fermentation. Thus, flocculation property of yeast is very important property from the standpoint of working efficiency of brewing process. That is, yeast having poor flocculation property does not sediment at the end of fermentation, and there is a problem that extraneous steps such as centrifugation are required for recover it. On the other hand, yeast having undesirably high flocculation property may sediment during fermentation, which can result in immature termination of fermentation. In that case, flavor and taste of resultant product are also seriously influenced. Accordingly, it is very important to use yeast having suitable flocculation property for production of desired alcoholic beverages.

Further, flocculation of yeast is actively studied, because efficiency is required especially in the field of industrial alcohol production (Novel agglutinative alcohol fermenting yeast, Japanese Examined Patent Publication (Kokoku) No. H6-36734) and wastewater treatment (Japanese Patent No. 3044284), as well as in the field of alcoholic beverage production.

FLO gene family (FLO1, FLO5, FLO8, FLO9, FLO10, FLO11) and SFL1, etc. have ever been recognized as genes responsible for flocculation property of yeast as a result of enormous quantity of investigations on flocculation property, which is of importance for industry, of yeast as stated above.

In the investigation, for example, property of FLO1 gene was analyzed at molecular level (Bidard et al., YEAST, 11(9), 809, 1995). Method for controlling flocculation property of beer yeast was investigated as well using Flo1p (Japanese Patent No. 3643404).

Method of controlling flocculation property of yeast by regulating expression of FLO8 gene, and method of judging flocculating property using, nucleotide sequence of FLO5 gene (International Publication No. WO01/040514), etc. are also reported.

On the other hand, some of yeast cell surface proteins specific to flocculating yeast are known as well However, knowledge of each proteins is not sufficient for research to control flocculation property of beer yeast

### DISCLOSURE OF INVENTION

Under the above situations, there has been a need for breeding yeasts having flocculation property suitable for production of desired alcoholic beverages to make high-efficiency production of alcoholic beverages possible by using a gene encoding a protein responsible for flocculation property of brewery yeast and said protein.

The present inventors made extensive studies to solve the above problems and as a result, succeeded in identifying and isolating a gene encoding a protein responsible for flocculation property of yeast from beer yeast. Moreover, the present inventors produced transformed yeast in which expression of the obtained gene was controlled to verify that flocculation property can be actually controlled, thereby completing the present invention.

Thus, the present invention relates to a gene as defined in the claims encoding a protein responsible for flocculation property of brewery yeast, to a protein as defined in the claims, to a transformed yeast in which the expression of said gene is controlled. Also disclosed is a method for controlling flocculation property using a yeast in which the expression of said gene is controlled, or the like. More specifically, the present invention provides the following polynucleotides, or a vector comprising said polynucleotide, a transformed yeast comprising said vector, a method for producing alcoholic beverages by using said transformed yeast, and the like.

1. A polynucleotide selected from the group consisting of:

(a) a polynucleotide comprising a polynucleotide consisting of the nucleotide sequence of SEQ ID NO:1;
(b) a polynucleotide comprising a polynucleotide encoding a protein consisting of the amino acid sequence of SEQ ID NO:2; and
(c) a polynucleotide comprising a polynucleotide encoding a protein having an amino acid sequence having 80% or higher identity with the amino acid sequence of SEQ ID NO:2, and said protein having an activity of imparting flocculation property to yeast.

2. The polynucleotide according to (1) selected from the group consisting of a polynucleotide comprising a polynucleotide encoding a protein having 90% or higher identity with the amino acid sequence of SEQ ID NO: 2, and having an activity of imparting flocculation property to yeast.

3. The polynucleotide according to (1) comprising a polynucleotide consisting of the nucleotide sequence of SEQ ID NO: 1.

4. The polynucleotide according to (1) comprising a polynucleotide encoding a protein consisting of the amino acid sequence of SEQ ID NO: 2.

5. A protein consisting of the amino acid sequence of SEQ ID NO: 2.

6. A vector containing the polynucleotide according to any one of (1) to (4).

7. A yeast comprising the vector according to (6).

8. A method for producing an alcoholic beverage by using the yeast according to (7).

9. The method according to (8) comprising: conducting fermentation for producing an alcoholic beverage using the yeast according to (7).

10. A method for assessing a test yeast for its flocculation property, comprising:

(i) using a primer or probe including all or part of the nucleotide sequence of SEQ ID NO: 1 or being complementary to all or part of the nucleotide sequence of SEQ ID NO: 1; or
(ii) culturing the test yeast; and measuring the expression level of the gene having the nucleotide sequence of SEQ ID NO: 1 and encoding a protein having an activity of imparting flocculation property to yeast.

11. A method for selecting a yeast, comprising: culturing test yeasts; quantifying the protein of (5) or measuring the expression level of the gene having the nucleotide sequence of SEQ ID NO: 1 and encoding a protein having an activity of imparting flocculation property to yeast; and selecting a test yeast having an amount of the protein or the gene expression level according to the flocculation property of interest.

According to the method for producing alcoholic beverages using transformed yeast of the present invention, alcoholic beverages can be produced highly-efficiently by using yeasts having flocculation property suitable for production of desired alcoholic beverages.

**BRIEF DESCRIPTION OF DRAWINGS**

Figure 1 shows the cell growth with time upon beer fermentation test. The horizontal axis represents fermentation time while the vertical axis represents optical density at 660 nm (OD660).
Figure 2 shows the extract (sugar) consumption with time upon beer fermentation test The horizontal axis represents fermentation time while the vertical axis represents apparent extract concentration (w/w%).
Figure 3 shows the expression profile of non-ScKRE9 gene in yeasts upon beer fermentation test. The horizontal axis represents fermentation time while the vertical axis represents the intensity of detected signal.
Figure 4 shows the result of flocculation property test of non-ScKRE9 disrupted strain.

The vertical axis represents Segmentation Index indicating Flocculation property.

## BEST MODES FOR CARRYING OUT THE INVENTION

The present inventors isolated and identified non-ScKRE9 gene encoding a protein responsible for flocculation property of brewery yeast based on the lager brewing yeast genome information mapped according to the method disclosed in Japanese Patent Application Laid-Open No. 2004-283169. The nucleotide sequence of the gene is represented by SEQ ID NO: 1. Further, an amino acid sequence of a protein encoded by the gene is represented by SEQ ID NO: 2.

### 1. Polynucleotide of the invention

First of all, the present invention provides (a) a polynucleotide comprising a polynucleotide of the nucleotide sequence of SEQ ID NO: 1; and (b) a polynucleotide comprising a polynucleotide encoding a protein of the amino acid sequence of SEQ ID NO:2. The polynucleotide can be DNA or RNA.

The target polynucleotide of the present invention is not limited to the polynucleotide encoding a protein responsible for flocculation property derived from lager brewing yeast described above and may include other polynucleotides encoding proteins having equivalent functions to said protein. Proteins with equivalent functions are disclosed herein, for example, (c) a protein of an amino acid sequence of SEQ ID NO:2 with one or more amino acids thereof being deleted, substituted, inserted and/or added and imparting flocculation property to yeast.

Such proteins include a protein consisting of an amino acid sequence of SEQ ID NO:2 with, for example, 1 to 100, 1 to 90, 1 to 80, 1 to 70, 1 to 60, 1 to 50, 1 to 40, 1 to 39, 1 to 38, 1 to 37, 1 to 36, 1 to 35, 1 to 34, 1 to 33, 1 to 32, 1 to 31, 1 to 30, 1 to 29, 1 to 28, 1 to 27, 1 to 26, 1 to 25, 1 to 24, 1 to 23, 1 to 22, 1 to 21, 1 to 20, 1 to 19, 1 to 18, 1 to 17, 1 to 16, 1 to 15, 1 to 14,1 to 13, 1 to 12, 1 to 11, 1 to 10, 1 to 9, 1 to 8, 1 to 7, 1 to 6 (1 to several amino acids), 1 to 5, 1 to 4, 1 to 3, 1 to 2, or 1 amino acid residues thereof being deleted, substituted, inserted and/or added and imparting flocculation property to yeast. In general, the number of deletions, substitutions, insertions, and/or additions is preferably smaller. In addition, in accordance with the present invention such proteins include (d) a protein having an amino acid sequence with about

80% or higher, 81% or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher identity with the amino acid sequence of SEQ ID NO:2, and imparting flocculation property to yeast. In general, the percentage identity is preferably higher.

Flocculation property of yeast may be measured, for example, by a method described in Japanese Patent Application Laid-Open No. H8-205890.

Furthermore, disclosed is (e) a polynucleotide comprising a polynucleotide which hybridizes to a polynucleotide consisting of a nucleotide sequence complementary to the nucleotide sequence of SEQ ID NO:1 under stringent conditions and which encodes a protein imparting flocculation property to yeast; and (f) a polynucleotide comprising a polynucleotide which hybridizes to a polynucleotide complementary to a nucleotide sequence of encoding a protein of SEQ ID NO:2 under stringent conditions, and which encodes a protein imparting flocculation property to yeast.

Herein, "a polynucleotide that hybridizes under stringent conditions" refers to nucleotide sequence, such as a DNA, obtained by a colony hybridization technique, a plaque hybridization technique, a southern hybridization technique or the like using all or part of polynucleotide of a nucleotide sequence complementary to the nucleotide sequence of SEQ ID NO:1 or polynucleotide encoding the amino acid sequence of SEQ ID NO:2 as a probe. The hybridization method may be a method described, for example, in MOLECULAR CLONING 3rd Ed., CURRENT PROTOCOLS IN MOLECULAR BIOLOGY, John Wiley & Sons 1987-1997, and so on.

The term "stringent conditions" as used herein may be any of low stringency conditions, moderate stringency conditions or high stringency conditions. "Low stringency conditions" are, for example, 5 x SSC, 5 x Denhardt's solution, 0.5% SDS, 50% formamide at 32°C. "Moderate stringency conditions" are, for example, 5 x SSC, 5 x Denhardt's solution, 0.5% SDS, 50% formamide at 42°C. "High stringency conditions" are, for example, 5 x SSC, 5 x Denhardt's solution, 0.5% SDS, 50% formamide at 50°C. Under these conditions, a polynucleotide, such as a DNA, with higher homology is expected to be obtained efficiently at higher temperature, although multiple factors are involved in hybridization stringency including temperature, probe concentration, probe length, ionic strength, time, salt concentration and others, and one skilled in the art may appropriately select these factors to realize similar stringency.

When a commercially available kit is used for hybridization, for example, Alkphos Direct Labeling Reagents (Amersham Pharmacia) may be used. In this case, according to the attached protocol, after incubation with a labeled probe overnight, the membrane is washed with a primary wash buffer containing 0.1% (w/v) SDS at 55°C, thereby detecting hybridized polynucleotide, such as DNA.

Other polynucleotides that can be hybridized include polynucleotides having about 60% or higher, about 70% or higher, 71% or higher, 72% or higher, 73% or higher, 74% or higher, 75% or higher, 76% or higher, 77% or higher, 78% or higher, 79% or higher, 80% or higher, 81% or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher or 99.9% or higher identity to polynucleotide encoding the amino acid sequence of SEQ ID NO:2 as calculated by homology search software, such as FASTA and BLAST using default parameters.

Identity between amino acid sequences or nucleotide sequences may be determined using algorithm BLAST by Karlin and Altschul (Proc. Natl. Acad. Sci. USA, 87: 2264-2268, 1990; Proc. Natl. Acad Sci. USA, 90: 5873, 1993). Programs called BLASTN and BLASTX based on BLAST algorithm have been developed (Altschul SF et al., J. Mol. Biol. 215: 403, 1990). When a nucleotide sequence is sequenced using BLASTN, the parameters are, for example, score = 100 and word length = 12. When an amino acid sequence is sequenced using BLASTX, the parameters are, for example, score = 50 and word length = 3. When BLAST and Gapped BLAST programs are used, default parameters for each of the programs are employed.

The polynucleotide disclosed herein can be (j) a polynucleotide encoding RNA having a nucleotide sequence complementary to a transcript of the polynucleotide (DNA) according to (3) above; (k) a polynucleotide encoding RNA that represses the expression of the polynucleotide (DNA) according to (3) above through RNAi effect; (1) a polynucleotide encoding RNA having an activity of specifically cleaving a transcript of the polynucleotide (DNA) according to (3) above; and (m) a polynucleotide encoding RNA that represses expression of the polynucleotide (DNA) according to (3) above through co-supression effect. These polynucleotides may be incorporated into a vector, which can be introduced into a cell for transformation to repress the expression of the polynucleotides (DNA) of (a) to (i) above. Thus, these polynucleotides may suitably be used when repression of the expression of the above DNA is preferable.

The phrase "polynucleotide encoding RNA having a nucleotide sequence complementary to the transcript of DNA" as used herein refers to so-called antisense DNA. Antisense technique is known as a method for repressing expression of a particular endogenous gene, and is described in various publications (see e.g., Hirajima and Inoue: New Biochemistry Experiment Course 2 Nucleic Acids IV Gene Replication and Expression (Japanese Biochemical Society Ed., Tokyo Kagaku Dozin Co., Ltd.) pp.319-347, 1993). The sequence of antisense DNA is preferably complementary to all or part of the endogenous gene, but may not be completely complementary as long as it can effectively repress the expression of the gene. The transcribed RNA has preferably 90% or higher, and more preferably 95% or higher complementarity to the transcript of the target gene. The length of the antisense DNA is at least 15 bases or more, preferably 100 bases or more, and more preferably 500 bases or more.

The phrase "polynucleotide encoding RNA that represses DNA expression through RNAi effect" as used herein refers to a polynucleotide for repressing expression of an endogenous gene through RNA interference (RNAi). The term "RNAi" refers to a phenomenon where when double-stranded RNA having a sequence identical or similar to the target gene sequence is introduced into a cell, the expressions of both the introduced foreign gene and the target endogenous gene are repressed. RNA as used herein includes, for example, double-stranded RNA that causes RNA interference of 21 to 25 base length, for example, dsRNA (double strand RNA), siRNA (small interfering RNA) or shRNA (short hairpin RNA). Such RNA may be locally delivered to a desired site with a delivery system such as liposome, or a vector that generates the double-stranded RNA described above may be used for local expression thereof Methods for producing or using such double-stranded RNA (dsRNA, siRNA or shRNA) are known from many publications (see, e.g., Japanese National Phase PCT Laid-open Patent Publication No. 2002-516062; US 2002/086356A; Nature Genetics, 24(2), 180-183, 2000 Feb.; Genesis, 26(4), 240-244, 2000 April; Nature, 407:6802, 319-20, 2002 Sep. 21; Genes & Dev., Vol.16, (8), 948-958, 2002 Apr.15; Proc. Natl. Acad. Sci. USA., 99(8), 5515-5520,2002 Apr. 16; Science, 296(5567), 550-553,2002 Apr. 19; Proc Natl. Acad. Sci. USA, 99:9, 6047-6052, 2002 Apr. 30; Nature Biotechnology, Vol.20 (5), 497-500, 2002 May; Nature Biotechnology, Vol. 20(5), 500-505, 2002 May; Nucleic Acids Res., 30:10, e46,2002 May 15).

The phrase "polynucleotide encoding RNA having an activity of specifically cleaving transcript of DNA" as used herein generally refers to a ribozyme. Ribozyme is an RNA molecule with a catalytic activity that cleaves a transcript of a target DNA and inhibits the function of that gene. Design of ribozymes can be found in various known publications (see, e.g., FEBS Lett. 228: 228, 1988; FEBS Lett. 239: 285, 1988; Nucl. Acids. Res. 17: 7059, 1989; Nature 323: 349, 1986; Nucl. Acids. Res. 19: 6751, 1991; Protein Eng 3: 733, 1990; Nucl. Acids Res. 19:3875, 1991; Nucl. Acids Res. 19: 5125, 1991; Biochem Biophys Res Commun 186: 1271, 1992). In addition, the phrase "polynucleotide encoding RNA that represses DNA expression through co-supression effect" refers to a nucleotide that inhibits functions of target DNA by "co-supression".

The term "co-supression" as used herein, refers to a phenomenon where when a gene having a sequence identical or similar to a target endogenous gene is transformed into a cell, the expressions of both the introduced foreign gene and the target endogenous gene are repressed. Design of polynucleotides having a co-supression effect can also be found in various publications (see, e.g., Smyth DR: Curr. Biol. 7: R793, 1997, Martienssen R: Curr. Biol. 6: 810, 1996).

## 2. Protein of the present invention

The present invention also provides proteins encoded by any of the polynucleotides (1) to (2) above. Disclosed is a protein that comprises an amino acid sequence of SEQ ID NO:2 with one or several amino acids thereof being deleted, substituted, inserted and/or added, and imparts flocculation property to yeast.

Such protein includes those having an amino acid sequence of SEQ ID NO:2 with amino acid residues thereof of the number mentioned above being deleted, substituted, inserted and/or added and imparting flocculation property to yeast. In addition, such protein includes those having homology as described above with the amino acid sequence of SEQ ID NO:2 and imparting flocculation property to yeast.

Such proteins may be obtained by employing site-directed mutation described, for example, in MOLECULAR CLONING 3rd Ed., CURRENT PROTOCOLS IN MOLECULAR BIOLOGY, Nuc. Acids. Res., 10: 6487 (1982), Proc. Natl. Acad Sci. USA 79: 6409 (1982), Gene 34: 315 (1985), Nuc. Acids. Res., 13:4431 (1985), Proc. Natl. Acad Sci. USA 82: 488 (1985).

Deletion, substitution, insertion and/or addition of one or more amino acid residues in an amino acid sequence of the protein of the invention means that one or more amino acid residues are deleted, substituted, inserted and/or added at any one or more positions in the same amino acid sequence. Two or more types of deletion, substitution, insertion and/or addition may occur concurrently.

Hereinafter, examples of mutually substitutable amino acid residues are enumerated. Amino acid residues in the same group are mutually substitutable. The groups are provided below.

Group A: leucine, isoleucine, norleucine, valine, norvaline, alanine, 2-aminobutanoic acid, methionine, o-methylserine, t-butylglycine, t-butylalanine, cyclohexylalanine; Group B: asparatic acid, glutamic acid, isoasparatic acid, isoglutamic acid, 2-aminoadipic acid, 2-aminosuberic acid; Grou C: asparagine, glutamine; Group D: lysine, arginine, ornithine, 2,4-diaminobutanoic acid, 2,3-diaminopropionic acid; Group E: proline, 3-hydroxyproline, 4-hydroxyproline; Group F: serine, threonine, homoserine; and Group G: phenylalanine, tyrosine.

The protein of the present invention may also be produced by chemical synthesis methods such as Fmoc method (fluorenylmethyloxycarbonyl method) and tBoc method (t-butyloxycarbonyl method). In addition, peptide synthesizers available from, for example, Advanced ChemTech, PerkinElmer, Pharmacia, Protein Technology Instrument, Synthecell-Vega, PerSeptive, Shimazu Corp. can also be used for chemical synthesis.

## 3. Vector of the invention and yeast transformed with the vector

The present invention then provides a vector comprising the polynucleotide described above. The vector of the present invention is directed to a vector including any of the polynucleotides described in (1) to (2) above.

Generally, the vector of the present invention may comprise an expression cassette including as components (x) a promoter that can transcribe in a yeast cell; (y) a polynucleotide described in any of (1) to (2) above that is linked to the promoter in sense or antisense direction; and (z) a signal that functions in the yeast with respect to transcription termination and polyadenylation of RNA molecule.

According to the present invention, in order to highly express the protein of the invention described above upon brewing alcoholic beverages (e.g., beer) described below, these polynucleotides are introduced in the sense direction to the promoter to promote expression of the polynucleotide (DNA) described in any of (1) to (2)above. Further, in order to repress the above protein of the invention upon brewing alcoholic beverages (e.g., beer) described below, these polynucleotides are introduced in the antisense direction to the promoter to repress the expression of the polynucleotide (DNA) described in any of (1) to (2) above.

In order to repress the above protein of the invention, the polynucleotide may be introduced into vectors such that the polynucleotide of any of the (j) to (m) is to be expressed. In the present invention, the target gene (DNA) may be disrupted to repress the expression of the DNA described above or the expression of the protein described above. A gene may be disrupted by adding or deleting one or more bases to or from a region involved in expression of the gene product in the target gene, for example, a coding region or a promoter region, or by deleting these regions entirely. Such disruption of gene may be found in known publications (see, e.g., Proc. Natl. Acad. Sci. USA, 76, 4951(1979) , Methods in Enzymology, 101, 202(1983), Japanese Patent Application Laid-Open No.6-253826).

Further, in the present invention, the expression level of a target gene can be controlled by introducing a mutation to a promoter or genetically altering a promoter by homologous recombination. Such mutation introducing method is described in Nucleic Acids Res. 29, 4238-250 (2001), and such alteration of a promoter is described in, for example, Appl Environ Microbiol.,72, 5266-5273 (2006).

A vector introduced in the yeast may be any of a multicopy type (YEp type), a single copy type (YCp type), or a chromosome integration type (YIp type). For example, YEp24 (J. R Broach et al., EXPERIMENTAL MANIPULATION OF GENE EXPRESSION, Academic Press, New York, 83, 1983) is known as a YEp type vector, YCp50 (M. D. Rose et al., Gene 60: 237, 1987) is known as a YCp type vector, and YIp5 (K. Struhl et al., Proc. Natl. Acad. Sci. USA, 76: 1035, 1979) is known as a YIp type vector, all of which are readily available.

Promoters/terminators for adjusting gene expression in yeast may be in any combination as long as they function in the brewery yeast and they are not influenced by constituents in fermentation broth. For example, a promoter of glyceraldehydes 3-phosphate dehydrogenase gene (TDH3), or a promoter of 3-phosphoglycerate kinase gene (PGK1) may be used. These genes have previously been cloned, described in detail, for example, in M. F. Tuite et al., EMBO J., 1, 603 (1982), and are readily available by known methods.

Since an auxotrophy marker cannot be used as a selective marker upon transformation for a brewery yeast, for example, a geneticin-resistant gene (G418r), a copper-resistant gene (CUP1) (Marin et al., Proc. Natl. Acad Sci. USA, 81, 337 1984) or a cerulenin-resistant gene (fas2m, PDR4) (Junji Inokoshi et al., Biochemistry, 64, 660, 1992; and Hussain et al., Gene, 101: 149, 1991, respectively) may be used.

A vector constructed as described above is introduced into a host yeast. Examples of the host yeast include any yeast that can be used for brewing, for example, brewery yeasts for beer, wine and sake. Specifically, yeasts such as genus *Saccharomyces* may be used. According to the present invention, a lager brewing yeast, for example, *Saccharomyces pastorianus* W34/70, etc., *Saccharomyces carlsbergensis* NCYC453 or NCYC456, etc., or *Saccharomyces cerevisiae* NBRC1951, NBRC1952, NBRC1953 or NBRC1954, etc., may be used. In addition, whisky yeasts such as *Saccharomyces cerevisiae* NCYC90, wine yeasts such as wine yeasts #1, 3 and 4 from the Brewing Society of Japan, and sake yeasts such as sake yeast #7 and 9 from the Brewing Society of Japan may also be used but not limited thereto. In the present invention, lager brewing yeasts such as *Saccharomyces pastorianus* may be used preferably.

A yeast transformation method may be a generally used known method. For example, methods that can be used include but not limited to an electroporation method (Meth. Enzym., 194: 182 (1990)), a spheroplast method (Proc. Natl. Acad Sci. USA, 75: 1929(1978)), a lithium acetate method (J. Bacteriology, 153: 163 (1983)), and methods described in Proc. Natl. Acad Sci. USA, 75: 1929 (1978), METHODS IN YEAST GENETICS, 2000 Edition: A Cold Spring Harbor Laboratory Course Manual.

More specifically, a host yeast is cultured in a standard yeast nutrition medium (e.g., YEPD medium (Genetic Engineering. Vol. 1, Plenum Press, New York, 117(1979)), etc.) such that OD600 nm will be 1 to 6. This culture yeast is collected by centrifugation, washed and pre-treated with alkali metal ion, preferably lithium ion at a concentration of about 1 to 2 M. After the cell is left to stand at about 30°C for about 60 minutes, it is left to stand with DNA to be introduced (about 1 to 20 $\mu$g) at about 30°C for about another 60 minutes. Polyethyleneglycol, preferably about 4,000 Dalton of polyethyleneglycol, is added to a final concentration of about 20% to 50%. After leaving at about 30°C for about 30 minutes, the cell is heated at about 42°C for about 5 minutes. Preferably, this cell suspension is washed with a standard yeast nutrition medium, added to a predetermined amount of fresh standard yeast nutrition medium and left to stand at about 30°C for about 60 minutes. Thereafter, it is seeded to a standard agar medium containing an antibiotic or the like as a selective marker to obtain a transformant.

Other general cloning techniques may be found, for example, in MOLECULAR CLONING 3rd Ed., and METHODS IN YEAST GENETICS, A LABORATORY MANUAL (Cold Spring Harbor Laboratory Press, Cold Spring Harbor, NY).

## 4. Method of producing alcoholic beverages according to the present invention and alcoholic beverages produced by the method

A yeast having flocculation property suitable for a target alcoholic beverages can be obtained by introducing the vector of the present invention or DNA fragments described above to a yeast suitable for brewing target alcoholic beverages to control expression level of the gene. Thus, alcoholic beverages can be produced highly-efficiently. That is to say, desired kind of alcoholic beverages can be produced highly-efficiently by controlling (elevating or reducing) flocculation property using yeasts into which the vector of the present invention was introduced described above, yeasts in which expression of the polynucleotide (DNA) of the present invention described above was regulated (promoted or suppressed) or yeasts selected by the yeast assessment method of the invention described below for fermentation to produce alcoholic beverages. The target alcoholic beverages include, for example, but not limited to beer, beer-taste beverages such as sparkling liquor (*happoushu*), wine, whisky, sake and the like. Further, alcohol for practical use such as alcohol for fuel is also included among them.

In order to produce these alcoholic beverages, a known technique can be used except that a brewery yeast obtained according to the present invention is used in the place of a parent strain. Since materials, manufacturing equipment, manufacturing control and the like may be exactly the same as the conventional ones, there is no need of increasing the cost for producing alcoholic beverages. Thus, according to the present invention, alcoholic beverages can be produced highly-efficiently using the existing facility without increasing the cost

## 5. Yeast assessment method of the invention

The present invention relates to a method for assessing a test yeast for its flocculation property by using a primer or a probe designed based on a nucleotide sequence of a gene having the nucleotide sequence of SEQ ID NO:1 and encoding a protein imparting flocculation property to yeast. General techniques for such assessment method is known and is described in, for example, WO01/040514, Japanese Laid-Open Patent Application No. H8-205900 or the like. This assessment method is described in below.

First, genome of a test yeast is prepared. For this preparation, any known-method such as Hereford method or potassium acetate method may be used (e.g., METHODS IN YEAST GENETICS, Cold Spring Harbor Laboratory Press, 130 (1990)). Using a primer or a probe designed based on a nucleotide sequence (preferably, ORF sequence) of the gene encoding a protein imparting flocculation property to yeast, the existence of the gene or a sequence specific to the gene is determined in the test yeast genome obtained. The primer or the probe may be designed according to a known technique.

Detection of the gene or the specific sequence may be carried out by employing a known technique. For example, a polynucleotide including part or all of the specific sequence or a polynucleotide including a nucleotide sequence complementary to said nucleotide sequence is used as one primer, while a polynucleotide including part or all of the sequence upstream or downstream from this sequence or a polynucleotide including a nucleotide sequence complementary to said nucleotide sequence, is used as another primer to amplify a nucleic acid of the yeast by a PCR method, thereby determining the existence of amplified products and molecular weight of the amplified products. The number of bases of polynucleotide used for a primer is generally 10 base pairs (bp) or more, and preferably 15 to 25 bp. In general, the number of bases between the primers is suitably 300 to 2000 bp.

The reaction conditions for PCR are not particularly limited but may be, for example, a denaturation temperature of 90 to 95°C, an annealing temperature of 40 to 60°C, an elongation temperature of 60 to 75°C, and the number of cycle of 10 or more. The resulting reaction product may be separated, for example, by electrophoresis using agarose gel to determine the molecular weight of the amplified product. This method allows prediction and assessment of the flocculation property of yeast as determined by whether the molecular weight of the amplified product is a size that contains the DNA molecule of the specific part. In addition, by analyzing the nucleotide sequence of the amplified product, the property may be predicted and/or assessed more precisely.

Moreover, in the present invention, a test yeast is cultured to measure an expression level of the gene encoding a protein imparting flocculation property to yeast having the nucleotide sequence of SEQ ID NO:1 to assess the test yeast for its flocculation property. In this case, the test yeast is cultured and then mRNA or a protein resulting from the gene encoding a protein imparting flocculation property to yeast is quantified. The quantification of mRNA or protein may be carried out by employing a known technique. For example, mRNA may be quantified, by Northern hybridization or quantitative RT-PCR, while protein may be quantified, for example, by Western blotting (CURRENT PROTOCOLS IN MOLECULAR BIOLOGY, John Wiley & Sons 1994-2003). Furthermore, test yeasts are cultured and expression levels of the gene encoding a protein imparting flocculation property to yeast having the nucleotide sequence of SEQ ID NO:1 are measured to select a test yeast with the gene expression level according to the target flocculation property, thereby selecting a yeast favorable for brewing desired alcoholic beverages. In addition, a reference yeast and a test yeast may be cultured so as to measure and compare the expression level of the gene in each of the yeasts, thereby selecting a favorable test yeast. More specifically, for example, a reference yeast and one or more test yeasts are cultured and an expression level of the gene having the nucleotide sequence of SEQ ID NO:1 is measured in each yeast. By selecting a test yeast with the gene expressed higher (i.e., expression level is enhanced) or lower (i.e., expression level is suppressed) than that in the reference yeast, a yeast suitable for brewing alcoholic beverages can be selected.

Alternatively, test yeasts are cultured and a yeast with a high or low flocculation property is selected, thereby selecting a yeast suitable for brewing desired alcoholic beverages.

In these cases, the test yeasts or the reference yeast may be, for example, a yeast introduced with the vector or DNA fragment of the invention, a yeast in which an expression of a polynucleotide (DNA) of the invention has been controlled, an artificially mutated yeast or a naturally mutated yeast. The flocculation property of yeast can be measured by, for example, a method described in Japanese Patent Application Laid-Open No. H8-205890. The mutation treatment may employ any methods including, for example, physical methods such as ultraviolet irradiation and radiation irradiation, and chemical methods associated with treatments with drugs such as EMS (ethylmethane sulphonate) and N-methyl-N-nitrosoguanidine (*see, e.g.,* Yasuji Oshima Ed., BIOCHEMISTRY EXPERIMENTS vol. 39, Yeast Molecular Genetic Experiments, pp. 67-75, JSSP).

In addition, examples of yeasts used as the reference yeast or the test yeasts include any yeasts that can be used for brewing, for example, brewery yeasts for beer, wine, sake and the like. More specifically, yeasts such as genus *Saccharomyces* may be used (*e.g., S. pastorianus, S. cerevisiae,* and *S. carlsbergensis*). According to the present invention, a lager brewing yeast, for example, *Saccharomyces pastorianus* W34/70; *Saccharomyces carlsbergensis* NCYC453 or NCYC456; or *Saccharomyces cerevisiae* NBRC1951, NBRC1952, NBRC1953 or NBRC1954, etc., may be used. Further, wine yeasts such as wine yeasts #1, 3 and 4 from the Brewing Society of Japan; and sake yeasts such as sake yeast #7 and 9 from the Brewing Society of Japan may also be used but not limited thereto. In the present invention, lager brewing yeasts such as *Saccharomyces pastorianus* may preferably be used. The reference yeast and the test yeasts may be selected from the above yeasts in any combination.

## EXAMPLES

Hereinafter, the present invention will be described in more detail with reference to working examples. The present invention, however, is not limited to the examples described below.

### Example 1: Cloning of Gene Encoding Protein Responsible for Flocculation Property of Yeast (nonScKRE9)

A gene encoding a protein responsible for flocculation property of brewery yeast (nonScKRE9) (SEQ ID NO: 1) was found as a result of a search utilizing the comparison database described in Japanese Patent Application Laid-Open No. 2004-283169. Based on the acquired nucleotide sequence information, primers nonScKRE9_F (SEQ ID NO: 3) and nonScKRE9_R (SEQ ID NO: 4) were designed to amplify the full-length of the gene. PCR was carried out using chromosomal DNA of a genome sequencing strain, Saccharomyces pastorianus Weihenstephan 34/70 (sometimes abbreviated as "W34/79 strain"), as a template to obtain DNA fragments including the full-length gene of nonScKRE9.

The nonScKRE9 gene fragments thus obtained were inserted into pCR2.1-TOPO vector (Invitrogen) by TA cloning. The nucleotide sequences of the nonScKRE9 gene were analyzed by Sanger's method (F. Sanger, Science, 214: 1215, 1981) to confirm the nucleotide sequence.

### Example 2: Analysis of Expression of nonScKRE9 Gene during Beer Fermentation

A beer fermentation test was conducted using a lager brewing yeast, Saccharomyces pastorianus W34/70.

| | |
|---|---|
| Wort extract concentration | 12.69% |
| Wort content | 70 L |
| Wort dissolved oxygen concentration | 8.6 ppm |
| Fermentation temperature | 15°C |
| Yeast pitching rate | $12.8\times10^6$ cells/mL |

The fermentation liquor was sampled over time, and the time-course changes in amount of yeast cell growth (Fig. 1) and apparent extract concentration (Fig. 2) were observed. Simultaneously, yeast cells were sampled to prepared mRNA, and the prepared mRNA was labeled with biotin and was hybridized to a beer yeast DNA microarray. The signal was detected using the GeneChip Operating system (GCOS; GeneChip Operating Software 1.0, manufactured by Affymetrix Co). Expression pattern of the nonScKRE9 gene is shown in Figure 3. This result confirmed the expression of the nonScKRE9 gene in the general beer fermentation.

### Example 3: Disruption of nonScKRE9 Gene

Fragments for gene disruption were prepared by PCR using a plasmid containing a drug resistance marker (pFA6a ($G418^r$), pAG25(natl) or pAG32 (hph)) as a template in accordance with a method described in literature (Goldstein et al., Yeast, 15, 1541 (1999)). Primers consisting of nonScKRE9_delta_for (SEQ ID NO. 5) and nonScKRE9_delta_rv (SEQ ID NO. 6) were used for the PCR primers.

A spore clone (W34/70-2) isolated from lager brewing yeast Saccharomyces pastorianus strain W34/70 was transformed with the fragments for gene disruption prepared as described above. Transformation was carried out according to the method described in Japanese Patent Application Laid-open No. H07-303475, and transformants were selected pn YPD plate.medium (1% yeast extract, 2% polypeptone, 2% glucose, 2% agar) containing 300 mg/L of geneticin, 50 mg/L of nourseothricin or 200 mg/L of hygromycin B.

### Example 4: Evaluation of Flocculation Property of non-ScKRE9 Disrupted Strain

Flocculation properties of the disrupted strain obtained in Example 3 and the parent strain (W34/70-2 strain) were evaluated by a method described below.

The yeasts were inoculated into 50 mL of glucose CM medium (3% glucose, 1% bacto peptone, 0.5% yeast

extract, 0.5% $KH_2PO_4$, 0.2% $MgSO_4.7H_2O$), then cultured statically at 30°C for 2 day.

Whole amount of the culture was transferred to 450 mL of glucose CM medium and cultured statically at 15°C for 4 days. The yeast cells were collected by centrifugation followed washing twice in cold water of 5°C. One point five grams (1.5 g) of the obtained wet yeast cells were accurately weighed, suspended in 20 mL of 0.1M acetate buffer containing 2mM Ca, and temperature equilibrated in an temperature controlled room at 15°C.

Subsequent measurement procedures were performed in a temperature controlled room at 15°C. The yeast suspension mixed for 30 seconds by vortex mixer was poured into a buret, then immediately 2 mL of the suspension was collected as a 0 min sample. After leaving 10 min at rest, 2 mL of the suspension was collected as a 10 min sample.

OD600 of the collected yeast suspensions were measured, respectively, and flocculation properties (Segmentation Index; SI) were calculated by formula below. The results are indicated in Figure 4. The values were averages of measurement of n = 2.

$$SI=(OD_{10min}-OD_{0min})/OD_{0min}*100$$

As indicated in Figure 4, SI = 117 in the disrupted strain shows that flocculation property of yeast was decreased by disruption of non-ScKRE9 by comparison with SI = 232 in the parent strain.

## Example 5: Construction of nonScKRE9-Highly Expressed Strain

The nonScKRE9/pCR2.1-TOPO described in Example 1 is digested with the restriction enzymes SacI and NotI to prepare a DNA fragment containing the entire length of the protein-encoding region. This fragment is ligated to pYCGPYNot treated with the restriction enzymes SacI and NotI, thereby constructing the nonScKRE9 high expression vector nonScKRE9/pYCGPYNot. pYCGPYNot is a YCp-type yeast expression vector. A gene inserted is highly expressed by the pyruvate kinase gene PYK1 promoter. The geneticin-resistant gene $G418^r$ is included as the selectable marker in the yeast, and the ampicillin-resistant gene $Amp^r$ as the selectable marker in Escherichia coli.

Using the high expression vector prepared by the above method, the strain Saccharomyces pasteurianus Weihenstephaner 34/70 is transformed by the method described in Japanese Patent Application Laid-open No. H07-303475. The transformants are selected on a YPD plate medium (1% yeast extract, 2% polypeptone, 2% glucose and 2% agar) containing 300 mg/L of geneticin.

Flocculation properties of the highly expressed strain obtained by the method described above and the parent strain (W34/70 strain) are evaluated by the same method as Example 4.

## INDUSTRIAL APPLICABILITY

The method of producing alcoholic beverages of the present invention may allow for highly-efficient production of alcoholic beverages by using a yeast having suitable for production of desired alcoholic beverages, because the flocculation property of yeast during fermentation can be controlled.

## Claims

**1.** A polynucleotide selected from the group consisting of:

(a) a polynucleotide comprising a polynucleotide consisting of the nucleotide sequence of SEQ ID NO:1;
(b) a polynucleotide comprising a polynucleotide encoding a protein consisting of the amino acid sequence of SEQ ID NO:2; and

(c) a polynucleotide comprising a polynucleotide encoding a protein having an amino acid sequence having 80% or higher identity with the amino acid sequence of SEQ ID NO:2, and said protein having an activity of imparting flocculation property to yeast.

**2.** The polynucleotide according to Claim 1 comprising a polynucleotide encoding a protein having 90% or higher identity with the amino acid sequence of SEQ ID NO: 2, and having an activity of imparting flocculation property to yeast.

**3.** The polynucleotide according to Claim 1 comprising a polynucleotide consisting of the nucleotide sequence of SEQ ID NO: 1.

**4.** The polynucleotide according to Claim 1 comprising a polynucleotide encoding a protein consisting of the amino acid sequence of SEQ ID NO: 2.

**5.** A protein consisting of the amino acid sequence of SEQ ID NO: 2.

**6.** A vector containing the polynucleotide according to any one of Claims 1 to 4.

**7.** A yeast comprising the vector according to Claim 6.

**8.** A method for producing an alcoholic beverage by using the yeast according to Claim 7.

**9.** The method according to claim 8 comprising: conducting fermentation for producing an alcoholic beverage using the yeast according to Claim 7.

**10.** A method for assessing a test yeast for its flocculation property, comprising:

(i) using a primer or probe including all or part of the nucleotide sequence of SEQ ID NO: 1 or being complementary to all or part of the nucleotide sequence of SEQ ID NO: 1; or
(ii) culturing the test yeast; and measuring the expression level of the gene having the nucleotide sequence of SEQ ID NO: 1 and encoding a protein having an activity of imparting flocculation property to yeast.

**11.** A method for selecting a yeast, comprising: culturing test yeasts; quantifying the protein of Claim 5 or measuring the expression level of the gene having the nucleotide sequence of SEQ ID NO: 1 and encoding a protein having an activity of imparting flocculation property to yeast; and selecting a test yeast having an amount of the protein or the gene expression level according to the flocculation property of interest.

## Patentansprüche

**1.** Polynucleotid ausgewählt aus der Gruppe bestehend aus:

(a) Polynucleotid, das ein Polynucleotid umfasst, das aus der Nucleotidsequenz der SEQ ID NO:1 besteht;
(b) Polynucleotid, das ein Polynucleotid umfasst, das ein Protein codiert, das aus der Aminosäuresequenz der SEQ ID NO:2 besteht; und
(c) Polynucleotid, das ein Polynucleotid umfasst, das ein Protein codiert, das eine Aminosäuresequenz hat, die eine Identität von 80% oder höher mit der Aminosäuresequenz der SEQ ID NO:2 hat, und wobei das Protein eine Aktivität hat, Hefe Flockungseigenschaft zu verleihen.

**2.** Polynucleotid nach Anspruch 1, das ein Polynucleotid umfasst, das ein Protein codiert, das eine Identität von 90% oder höher mit der Aminosäuresequenz der SEQ ID NO:2 hat, und eine Aktivität hat, Hefe Flockungseigenschaft zu verleihen.

**3.** Polynucleotid nach Anspruch 1, das ein Polynucleotid umfasst, das aus der Nucleotidsequenz der SEQ ID NO:1 besteht.

**4.** Polynucleotid nach Anspruch 1, das ein Polynucleotid umfasst, das ein Protein codiert, das aus der Aminosäuresequenz der SEQ ID NO:2 besteht.

**5.** Protein, das aus der Aminosäuresequenz der SEQ ID NO:2 besteht.

**6.** Vektor, der das Polynucleotid nach einem der Ansprüche 1 bis 4 enthält.

**7.** Hefe, die den Vektor nach Anspruch 6 umfasst.

**8.** Verfahren zur Herstellung eines alkoholischen Getränks unter Verwendung der Hefe nach Anspruch 7.

**9.** Verfahren nach Anspruch 8, umfassend das Durchführen einer Fermentation zur Herstellung eines alkoholischen Getränks unter Verwendung der Hefe nach Anspruch 7.

**10.** Verfahren zur Beurteilung der Flockungseigenschaft einer Test-Hefe, umfassend:

(i) das Verwenden eines Primers oder einer Sonde, der/die die gesamte Nucleotidsequenz der SEQ ID NO:1 oder einen Teil davon enthält, oder, der/die komplementär zu der Nucleotidsequenz der SEQ ID NO:1 oder einem Teil davon ist; oder
(ii) das Züchten der Test-Hefe; und das Messen des Expressionsspiegels des Gens, das die Nucleotidsequenz der SEQ ID NO:1 hat und ein Protein codiert, das eine Aktivität hat, Hefe Flockungseigenschaft zu verleihen.

**11.** Verfahren zur Auswahl einer Hefe, umfassend: das Züchten von Test-Hefen; das Quantifizieren des Proteins nach Anspruch 5 oder das Messen des Expressionsspiegels des Gens, das die Nucleotidsequenz der SEQ ID NO:1 hat und ein Protein codiert, das eine Aktivität hat, Hefe Flockungseigenschaft zu verleihen; und das Auswählen einer Test-Hefe, die eine Menge des Proteins oder des Gen-Expressionsspiegels entsprechend der Flockungseigenschaft von Interesse aufweist.

**Revendications**

**1.** Polynucléotide sélectionné dans le groupe consistant en :

(a) un polynucléotide comprenant un polynucléotide consistant en la séquence de nucléotides de SEQ ID NO: 1 ;
(b) un polynucléotide comprenant un polynucléotide codant pour une protéine consistant en la séquence d'acides aminés de SEQ ID NO: 2 ; et
(c) un polynucléotide comprenant un polynucléotide codant pour une protéine ayant une séquence d'acides aminés présentant 80 % d'identité ou plus avec la séquence d'acides aminés de SEQ ID NO: 2, et ladite protéine ayant une activité qui confère une propriété de floculation à une levure.

**2.** Polynucléotide selon la revendication 1, comprenant un polynucléotide codant pour une protéine présentant 90 % d'identité ou plus avec la séquence d'acides aminés de SEQ ID NO:2, et ayant une activité qui confère une propriété de floculation à une levure.

**3.** Polynucléotide selon la revendication 1, comprenant un polynucléotide consistant en la séquence de nucléotides de SEQ ID NO: 1.

**4.** Polynucléotide selon la revendication 1, comprenant un polynucléotide codant pour une protéine consistant en la séquence d'acides aminés de SEQ ID NO: 2.

**5.** Protéine consistant en la séquence d'acides aminés de SEQ ID NO: 2.

**6.** Vecteur contenant le polynucléotide selon l'une quelconque des revendications 1 à 4.

**7.** Levure comprenant le vecteur selon la revendication 6.

**8.** Procédé de production d'une boisson alcoolisée en utilisant la levure selon la revendication 7.

**9.** Procédé selon la revendication 8, comprenant : la réalisation d'une fermentation afin de produire une boisson alcoolisée en utilisant la levure selon la revendication 7.

**10.** Procédé d'évaluation d'une levure de test pour sa propriété de floculation, comprenant :

(i) l'utilisation d'une amorce ou d'une sonde qui comprend l'intégralité ou une partie de la séquence de nucléotides de SEQ ID NO: 1 ou qui est complémentaire à l'intégralité ou à une partie de la séquence de nucléotides de SEQ ID NO: 1, ou
(ii) la mise en culture de la levure de test ; et la mesure du taux d'expression du gène ayant la séquence de nucléotides de SEQ ID NO: 1 et codant pour une protéine ayant une activité qui confère une propriété de floculation à une levure.

**11.** Procédé de sélection d'une levure, comprenant : la mise en culture de levures de test ; la quantification de la protéine selon la revendication 5 ou la mesure du taux d'expression du gène ayant la séquence de nucléotides de SEQ ID NO: 1 et codant pour une protéine ayant une activité qui confère une propriété de floculation à une levure ; et la sélection d'une levure de test présentant une quantité de taux d'expression de la protéine ou du gène selon la propriété de floculation d'intérêt.

FIG. 1

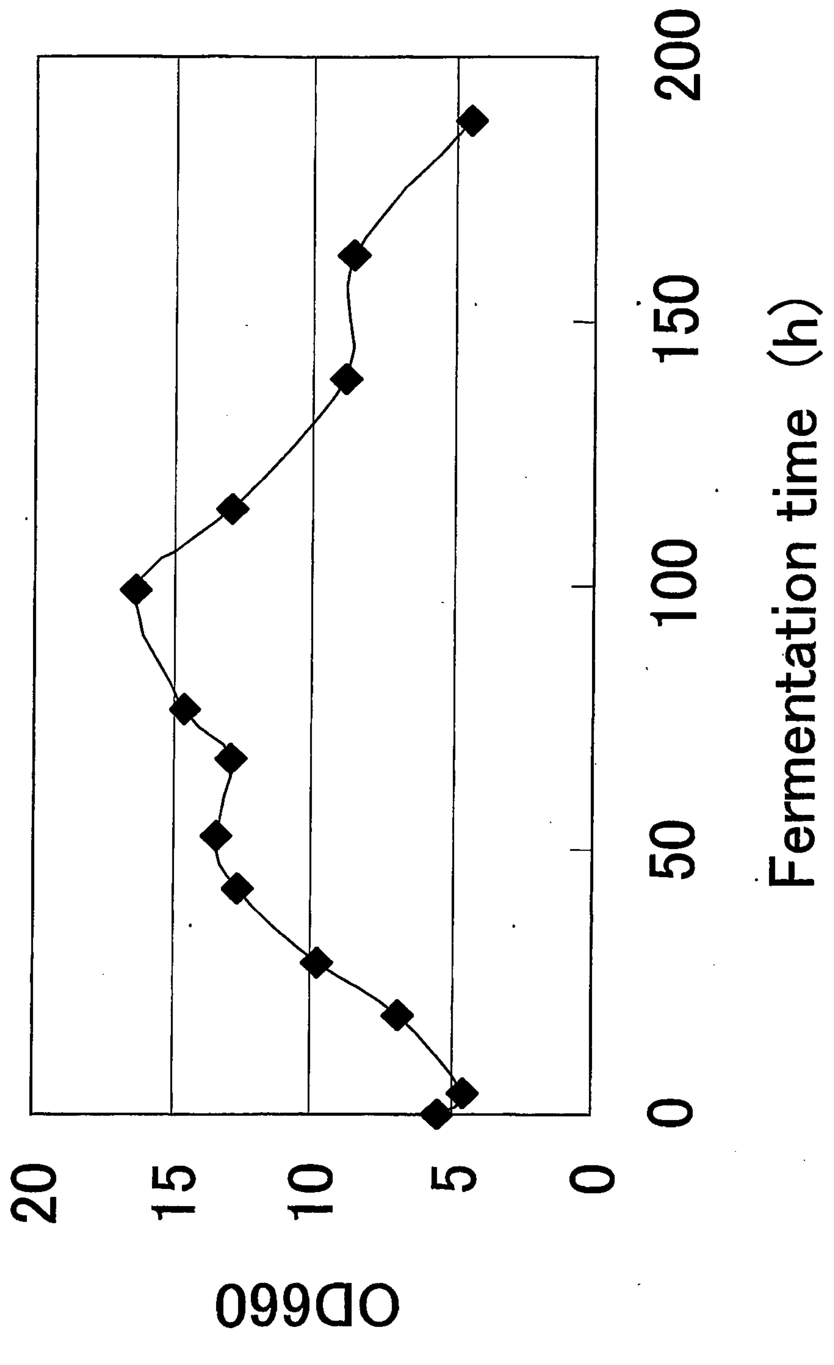

FIG. 2

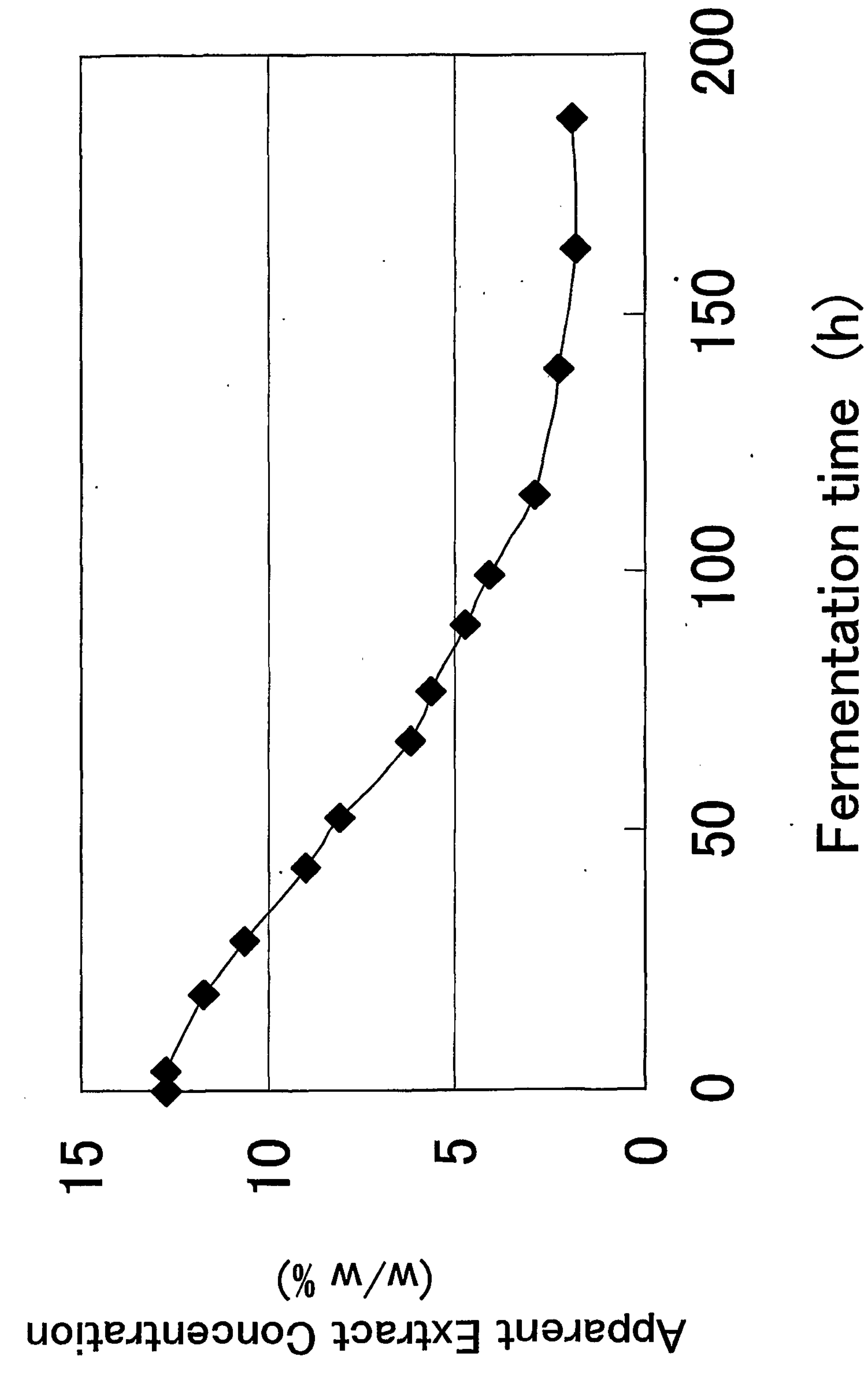

Apparent Extract Concentration
(w/w %)
15
10
5
0
0
50
100
150
200
Fermentation time (h)

FIG. 3

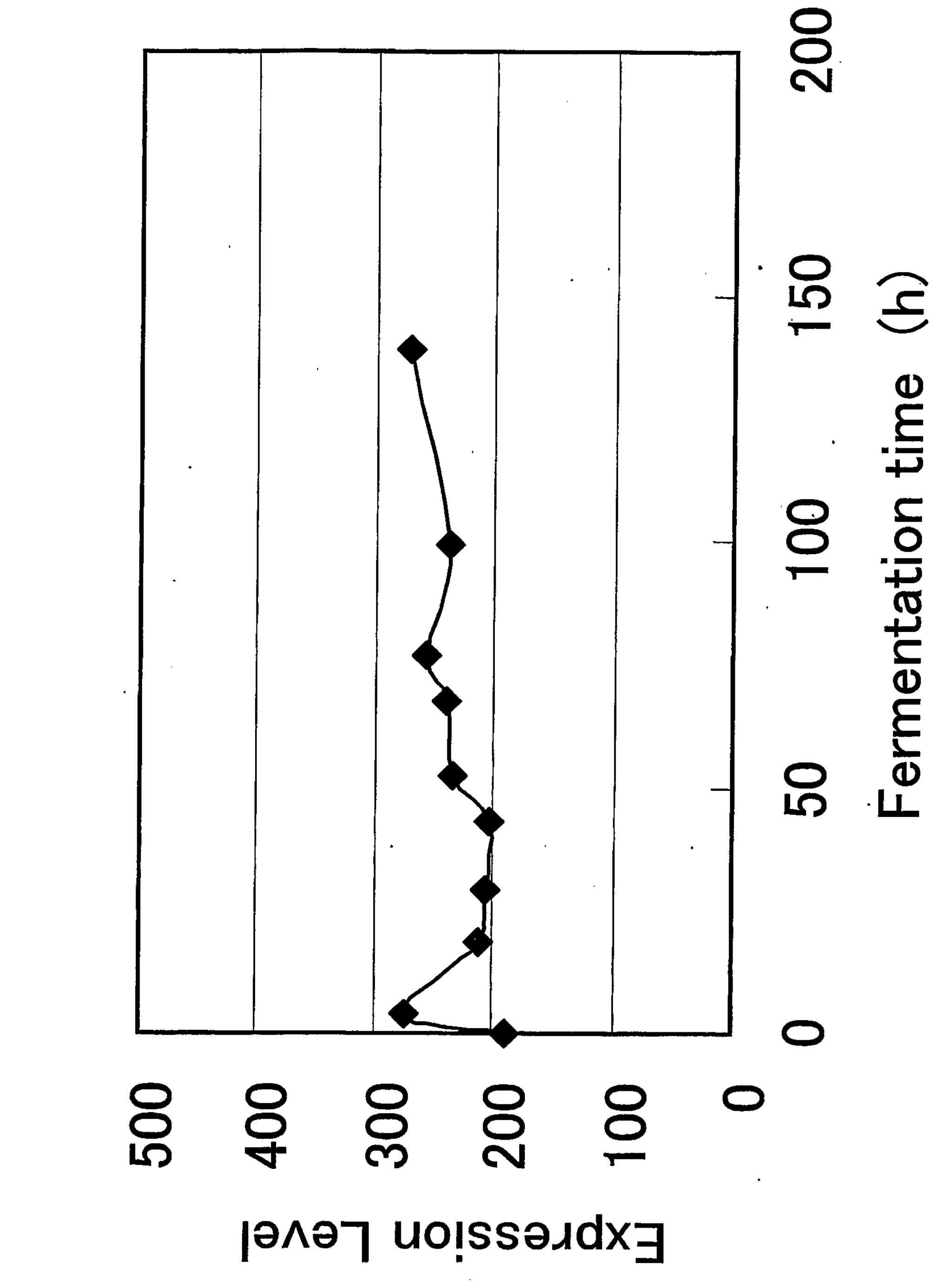
Expression Level
500
400
300
200
100
0
0
50
100
150
200
Fermentation time (h)

FIG. 4

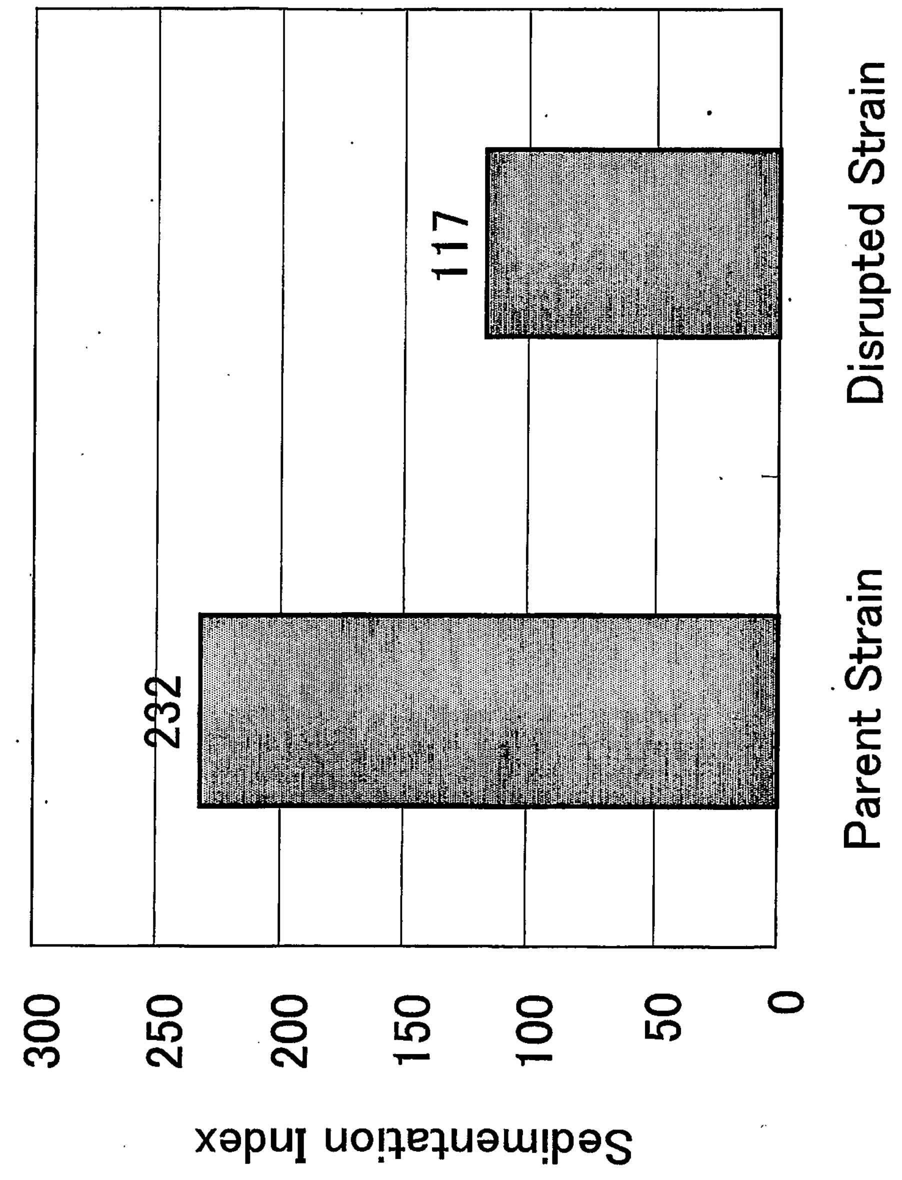

Sedimentation Index
300
250
200
150
100
50
0
232
117
Parent Strain
Disrupted Strain

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H636734 B
- JP 3044284 B
- JP 3643404 B
- WO 01040514 A
- JP 2004283169 A
- JP H8205890 B
- JP 2002516062 PCT
- US 2002086356 A
- JP 6253826 A
- JP H8205900 B
- JP H07303475 B
- JP 2006053938 A


### Non-patent literature cited in the description


- **BIDARD et al.** *YEAST,* 1995, vol. 11 (9), 809
- CURRENT PROTOCOLS IN MOLECULAR BIOLOGY. MOLECULAR CLONING. John Wiley & Sons, 1987
- **KARLIN ; ALTSCHUL.** *Proc. Natl. Acad. Sci. USA,* 1990, vol. 87, 2264-2268
- *Proc. Natl. Acad Sci. USA,* 1993, vol. 90, 5873
- **ALTSCHUL SF et al.** *J. Mol. Biol.,* 1990, vol. 215, 403
- **HIRAJIMA ; INOUE.** New Biochemistry Experiment Course 2 Nucleic Acids IV Gene Replication and Expression. Tokyo Kagaku Dozin Co., Ltd, 1993, 319-347
- *Nature Genetics,* February 2000, vol. 24 (2), 180-183
- *Genesis,* April 2000, vol. 26 (4), 240-244
- *Nature,* 21 September 2002, vol. 407 (6802), 319-20
- *Genes & Dev.,* 15 April 2002, vol. 16 (8), 948-958
- *Proc. Natl. Acad. Sci. USA.,* 16 April 2002, vol. 99 (8), 5515-5520
- *Science,* 19 April 2002, vol. 296 (5567), 550-553
- *Proc Natl. Acad. Sci. USA,* 30 April 2002, vol. 99 (9), 6047-6052
- *Nature Biotechnology,* May 2002, vol. 20 (5), 497-500
- *Nature Biotechnology,* May 2002, vol. 20 (5), 500-505
- *Nucleic Acids Res.,* 15 May 2002, vol. 30 (10), 46
- *FEBS Lett.,* 1988, vol. 228, 228
- *FEBS Lett.,* 1988, vol. 239, 285
- *Nucl. Acids. Res.,* 1989, vol. 17, 7059
- *Nature,* 1986, vol. 323, 349
- *Nucl. Acids. Res.,* 1991, vol. 19, 6751
- *Protein Eng,* 1990, vol. 3, 733
- *Nucl. Acids Res.,* 1991, vol. 19, 3875
- *Nucl. Acids Res.,* 1991, vol. 19, 5125
- *Biochem Biophys Res Commun,* 1992, vol. 186, 1271
- *Smyth DR: Curr. Biol.,* 1997, vol. 7, R793
- **MARTIENSSEN R.** *Curr. Biol.,* 1996, vol. 6, 810
- CURRENT PROTOCOLS IN MOLECULAR BIOLOGY. MOLECULAR CLONING
- *Nuc. Acids. Res.,* 1982, vol. 10, 6487
- *Proc. Natl. Acad Sci. USA,* 1982, vol. 79, 6409
- *Gene,* 1985, vol. 34, 315
- *Nuc. Acids. Res.,* 1985, vol. 13, 4431
- *Proc. Natl. Acad Sci. USA,* 1985, vol. 82, 488
- *Proc. Natl. Acad. Sci. USA,* 1979, vol. 76, 4951
- *Methods in Enzymology,* 1983, vol. 101, 202
- *Nucleic Acids Res.,* 2001, vol. 29, 4238-250
- *Appl Environ Microbiol.,* 2006, vol. 72, 5266-5273
- **J. R BROACH et al.** EXPERIMENTAL MANIPULATION OF GENE EXPRESSION. Academic Press, 1983, 83
- **M. D. ROSE et al.** *Gene,* 1987, vol. 60, 237
- **K. STRUHL et al.** *Proc. Natl. Acad. Sci. USA,* 1979, vol. 76, 1035
- **M. F. TUITE et al.** *EMBO J.,* 1982, vol. 1, 603
- **MARIN et al.** *Proc. Natl. Acad Sci. USA,* 1984, vol. 81, 337
- **JUNJI INOKOSHI et al.** *Biochemistry,* 1992, vol. 64, 660
- **HUSSAIN et al.** *Gene,* 1991, vol. 101, 149
- *Meth. Enzym.,* 1990, vol. 194, 182
- *Proc. Natl. Acad Sci. USA,* 1978, vol. 75, 1929
- *J. Bacteriology,* 1983, vol. 153, 163
- METHODS IN YEAST GENETICS. A Cold Spring Harbor Laboratory, 2000
- YEPD medium. Genetic Engineering. Plenum Press, 1979, vol. 1, 117

- MOLECULAR CLONING. METHODS IN YEAST GENETICS, A LABORATORY MANUAL. Cold Spring Harbor Laboratory Press
- METHODS IN YEAST GENETICS. Cold Spring Harbor Laboratory Press, 1990, 130
- **WESTERN.** CURRENT PROTOCOLS IN MOLECULAR BIOLOGY. John Wiley & Sons, 1994
- Yeast Molecular Genetic Experiments. BIOCHEMISTRY EXPERIMENTS. vol. 39, 67-75
- **F. SANGER.** *Science,* 1981, vol. 214, 1215

SEQUENCE LISTING

<110> Suntory Limited

<120> Gene encoding protein responsible for flocculation property of yeast and use thereof

<130> PCT06-0139

<150> JP2006-53938
<151> 2006-02-28

<160> 6

<170> PatentIn version 3.3

<210> 1
<211> 834
<212> DNA
<213> Saccharomyces sp.

<400> 1

```
atgcgtttac caacaggctc cattttatac gcttgggtgt ttttaatcac atgtgttctg     60
ggtgatgtaa acaccgtttc tccattagcc ggagccaaat tcagcccaag cggtggtaca    120
gtctctattc ccgtcaaatg gatggataat ggtgcgtatc ccccattaag caaaatttca    180
tactacacac tcagtctttg tactgggccc aacaatgaca ttaattgtat agggacaatt    240
gccacacagg tcaaacccag tgctttggct caggatcaag atggtgttta ctcttacaca    300
gctacttttc cctcgacttt aactggtaac ggtcaatatt atattcaagt tttcgcatgg    360
gtgaataatc aaggttctac cacccattat acgccaagat tccagttgac atccatggga    420
ggtactacag cttatacata cagtgactcg gcggcgccca ccccacagac ctccatccaa    480
actaccacta cgaataccgc acaagcatct tccatcgata gtcgttcatt tactgttcca    540
tacaccaaac aaaccggtac ttcacgtttc gctcccatgc aaatgcaacc tatgaccaag    600
gttaccgcta cgacatggac aagaaaatac gctaccagtg cagtgacata cttttctact    660
ttcgggaaat tgccccagca agaaactact ctaactccag gttggtctta ttcgatatct    720
tctggggtga actacgccac tcctgcagcc atgccatccg ataatggcgg ctggtataaa    780
ccatcgaaga gattatcttt gtctgccaga aagatgaatt tgagaaaagt ataa          834
```

<210> 2
<211> 277
<212> PRT
<213> Saccharomyces sp.

<400> 2

Met Arg Leu Pro Thr Gly Ser Ile Leu Tyr Ala Trp Val Phe Leu Ile
1               5                   10                  15
Thr Cys Val Leu Gly Asp Val Asn Thr Val Ser Pro Leu Ala Gly Ala
            20                  25                  30
Lys Phe Ser Pro Ser Gly Gly Thr Val Ser Ile Pro Val Lys Trp Met
        35                  40                  45
Asp Asn Gly Ala Tyr Pro Pro Leu Ser Lys Ile Ser Tyr Tyr Thr Leu
    50                  55                  60
Ser Leu Cys Thr Gly Pro Asn Asn Asp Ile Asn Cys Ile Gly Thr Ile
65                  70                  75                  80
Ala Thr Gln Val Lys Pro Ser Ala Leu Ala Gln Asp Gln Asp Gly Val
                85                  90                  95
Tyr Ser Tyr Thr Ala Thr Phe Pro Ser Thr Leu Thr Gly Asn Gly Gln
            100                 105                 110
Tyr Tyr Ile Gln Val Phe Ala Trp Val Asn Asn Gln Gly Ser Thr Thr
        115                 120                 125
His Tyr Thr Pro Arg Phe Gln Leu Thr Ser Met Gly Gly Thr Thr Ala
    130                 135                 140
Tyr Thr Tyr Ser Asp Ser Ala Ala Pro Thr Pro Gln Thr Ser Ile Gln
145                 150                 155                 160
Thr Thr Thr Thr Asn Thr Ala Gln Ala Ser Ser Ile Asp Ser Arg Ser
                165                 170                 175
Phe Thr Val Pro Tyr Thr Lys Gln Thr Gly Thr Ser Arg Phe Ala Pro
            180                 185                 190
Met Gln Met Gln Pro Met Thr Lys Val Thr Ala Thr Thr Trp Thr Arg
        195                 200                 205
Lys Tyr Ala Thr Ser Ala Val Thr Tyr Phe Ser Thr Phe Gly Lys Leu
    210                 215                 220
Pro Gln Gln Glu Thr Thr Leu Thr Pro Gly Trp Ser Tyr Ser Ile Ser
225                 230                 235                 240
Ser Gly Val Asn Tyr Ala Thr Pro Ala Ala Met Pro Ser Asp Asn Gly
                245                 250                 255
Gly Trp Tyr Lys Pro Ser Lys Arg Leu Ser Leu Ser Ala Arg Lys Met
            260                 265                 270
Asn Leu Arg Lys Val

275

<210> 3
<211> 40
<212> DNA
<213> Artificial

<220>
<223> Primer

<400> 3
gagctcatag cggccatgcg tttaccaaca ggctccattt 40

<210> 4
<211> 42
<212> DNA
<213> Artificial

<220>
<223> Primer

<400> 4
ggatcctatg cggccgctct gtttgctttt tttaaccttt ct 42

<210> 5
<211> 70
<212> DNA
<213> Artificial Sequence

<220>
<223> Primer

<400> 5

ttcccgccac atccttccac gaattcaaca gcagcgacag atcatagata ccttgacagt 60
cttgacgtgc 70

<210> 6
<211> 70
<212> DNA
<213> Artificial Sequence

<220>
<223> Primer

<400> 6

aataataata atagaaatag tagtgatgat agcagatatc tgcctactca cgcacttaac 60
ttcgcatctg 70